# EUROPÄISCHE PATENTSCHRIFT

(11) **EP 2 425 806 B1**
(45) Veröffentlichungstag und Bekanntmachung des Hinweises auf die Patenterteilung: **12.11.2014**
(21) Anmeldenummer: 10401160.6
(22) Anmeldetag: 07.09.2010
(51) Int. Cl.: A61G 9/02, A61L 2/18, A61L 2/07, A61L 2/24

(54) **Verfahren zur Reinigung von Steckbecken**
Cleaning method for bedpans
Procédé de nettoyage pour des bassins de lit

(43) Veröffentlichungstag der Anmeldung: 07.03.2012
(73) Patentinhaber: Miele & Cie. KG, 33332 Gütersloh (DE)
(72) Erfinder: Dittert, Jörg, 33613 Bielefeld (DE); Hemkendreis, Oliver, 33428 Harsewinkel (DE); Herbst, Christof, 33442 Herzebrock-Clarholz (DE); Horstmann, Peter, 33332 Gütersloh (DE); Scheele, Roland, 33613 Bielefeld (DE); Schilling, Olaf, 49163 Bohmte (DE)

(56) Entgegenhaltungen:
- EP-A1- 0 738 545
- DE-A1- 2 460 065
- DE-A1- 19 838 180
- DE-A1-102005 022 634

## Beschreibung

Die Erfindung betrifft ein Verfahren zur Reinigung von Steckbecken, Urinflaschen als Spülguter, bei dem eine mechanische Reinigung, anschließend eine chemische Reinigung und abschließend eine thermische Desinfektion durchgeführt wird.

Verfahren der vorgenannten Art sind aus dem Stand der Technik an sich bekannt, weshalb es keines gesonderten druckschriftlichen Nachweises an dieser Stelle bedarf.

Bei der Nutzung von Steckbecken, Urinflaschen kommt es insbesondere aufgrund von Medikamentenrückständen im Stuhlgang häufig zu stark anhaftenden Verschmutzungen. Für eine wunschgemäße Reinigung ist es deshalb oftmals erforderlich, trotz Einsatz von Reinigungsmitteln von Hand nachzureinigen. Alternativ ist das absolvierte Spülprogramm zu wiederholen.

Aus hygienischen Gründen arbeiten vorbekannte Vorrichtungen zur Reinigung von Steckbecken, Urinflaschen und/oder dergleichen nach dem Durchflussprinzip. Anders als bei herkömmlichen Spülmaschinen für den Hausgebrauch erfolgt also keine Umwälzung des Spülwassers. Das Spülwasser wird vielmehr gegebenenfalls unter Druck auf das zu reinigende Spülgut aufgebracht, von wo aus es dann über einen Siphon in die Abwasserleitung gelangt. Die Einwirkzeit für ein dem Reinigungswasser unter Umständen zugesetztes Reinigungsmittel ist deshalb kurz. Stärkere Verschmutzungen können deshalb auch nicht unter massivem Einsatz von Reinigungsmittel wirkungsvoll entfernt werden, weshalb oft auf eine manuelle Nachreinigung nicht verzichtet werden kann.

EP0738545 offenbart ein Verfahren zum Betrieb eines Wasch- und Sterilisierapparats nach dem Durchflussprinzip gemäß dem Oberbegriff des Anspruchs 1.

Aus der DE102005022634A1 und der DE2460065A1 sind darüber hinaus auch Verfahren zur Reinigung von Steckbecken, Urinflaschen und/oder dergleichen Spülgüter bekannt, bei denen nach einem ersten Entleerungs- bzw. Vorspülschritt die eigentliche Reinigung in einem Umwälzschritt erfolgt.

Die Aufgabe der Erfindung ist es, ein Verfahren zur Reinigung von insbesondere Steckbecken, Urinflaschen und/oder dergleichen Spülgüter vorzuschlagen, dass auch stark anhaftende Verschmutzungen entfernt, so dass auf manuelle Nachreinigungen möglichst verzichtet werden kann.

Zur Lösung dieser Aufgabe wird mit der Erfindung ein Verfahren zur Reinigung von Steckbecken, Urinflaschen gemäß Anspruch 1 vorgeschlagen.

Das erfindungsgemäße Verfahren zeichnet sich durch den Verfahrensschritt der chemischen Reinigung aus, welcher Verfahrensschritt dreistufig durchgeführt wird. In einer ersten Reinigungsstufe wird das zu reinigende Spülgut mit einem Gemisch aus Wasser und einem chemischen Reinigungsmittel beaufschlagt. Die Zufuhr an Wasser und/oder Reinigungsmittel wird dann gestoppt. Das auf dem Spülgut befindliche Gemisch aus Wasser und einem chemischen Reinigungsmittel kann alsdann einwirken und die vom Spülgut zu entfernenden Verschmutzungen aufweichen und ablösen. Dieser Einwirkvorgang erfolgt für eine vorgebbare Zeitspanne, die sich bevorzugterweise in Abhängigkeit des Verschmutzungsgrads des zu reinigenden Spülgutes einstellen lässt.

Nach Beendigung des Einwirkvorgangs erfolgt in einer dritten Stufe ein Abspülen des Spülguts, was mit frischem Wasser durchgeführt wird. Bei dem Frischwasser kann es sich um erwärmtes Frischwasser handeln.

Aufgrund des dreistufig ausgebildeten Verfahrensschrittes der chemischen Reinigung ist es möglich, eine Einwirkzeit für das chemische Reinigungsmittel vorzusehen, wodurch es gestattet ist, die Reinigungswirkung des Reinigungsmittels nutzen zu können, obgleich das gesamte Verfahren nach dem Durchflussprinzip arbeitet. Die nach der erfindungsgemäßen Verfahrensdurchführung vorgesehene Einwirkzeit gestattet es, auch hartnäckige Verschmutzungen ablösen zu können, so dass in der Konsequenz ein verbessertes Reinigungsergebnis erzielt werden kann. Dabei wird im Unterschied zu den aus dem Stand der Technik bekannten Verfahren der Einsatz von Ressourcen erheblich geschont, insbesondere mit Blick auf das einzusetzende Frischwasser. Darüber hinaus kann der Einsatz von Chemikalien auf ein Minimum reduziert werden, was aus umwelttechnischen Gründen von Vorteil ist.

Die Einwirkzeit kann bevorzugterweise vorgegeben werden. Dabei kann eine solche Vorgabe entweder benutzerseitig oder automatisiert erfolgen. Eine automatisierte Vorgabe lässt sich sowohl zeitgesteuert als auch unter Einsatz entsprechender Sensoren realisieren, die den Verschmutzungsgrad des Spülgutes detektieren können. Je nach festgestelltem Verschmutzungsgrad wird dann die Einwirkzeit entsprechend eingestellt.

Die einzelnen Behandlungsstufen des Verfahrensschrittes der chemischen Reinigung können mehrfach aufeinander nachfolgend durchgeführt werden. So kann insbesondere eine Wiederholung des gesamten Verfahrensschrittes der chemischen Reinigung vorgesehen sein. Dies kann insbesondere bei besonders stark anhaftenden Verschmutzungen von Nöten sein.

Gemäß einem weiteren Merkmal der Erfindung ist vorgesehen, dass zur Herstellung des Gemisches aus Wasser und einem Reinigungsmittel ein Wassertank dient, in den zunächst eine vorgebbare Menge eines Reinigungsmittels und anschließend eine vorgebbare Menge an Wasser eingefüllt wird. Auf diese Weise kann ein vorbestimmtes Mischungsverhältnis exakt eingestellt werden. Dabei kann das Mischungsverhältnis in Abhängigkeit der abzulösenden Verschmutzungen eingestellt werden.

Während der Verfahrensstufe des Einwirkens kann der Wassertank gemäß einem weiteren Merkmal der Erfindung mit Frischwasser befüllt werden. Für den nachfolgenden Schritt des Abspülens steht das Frischwasser im Wassertank dann bereits bereit, so dass ohne unnötigen Zeitverzug eine Verfahrensabwicklung durchgeführt werden kann.

Alle Merkmale und Vorteile der Erfindung ergeben sich aus der nachfolgenden Beschreibung anhand der Figuren. Dabei zeigen
- Fig. 1: in schematisch perspektivischer Darstellung eine Spüleinrichtung,
- Fig. 2: eine Spüleinrichtung nach Fig. 1 in teilgeschnittener Seitenansicht;

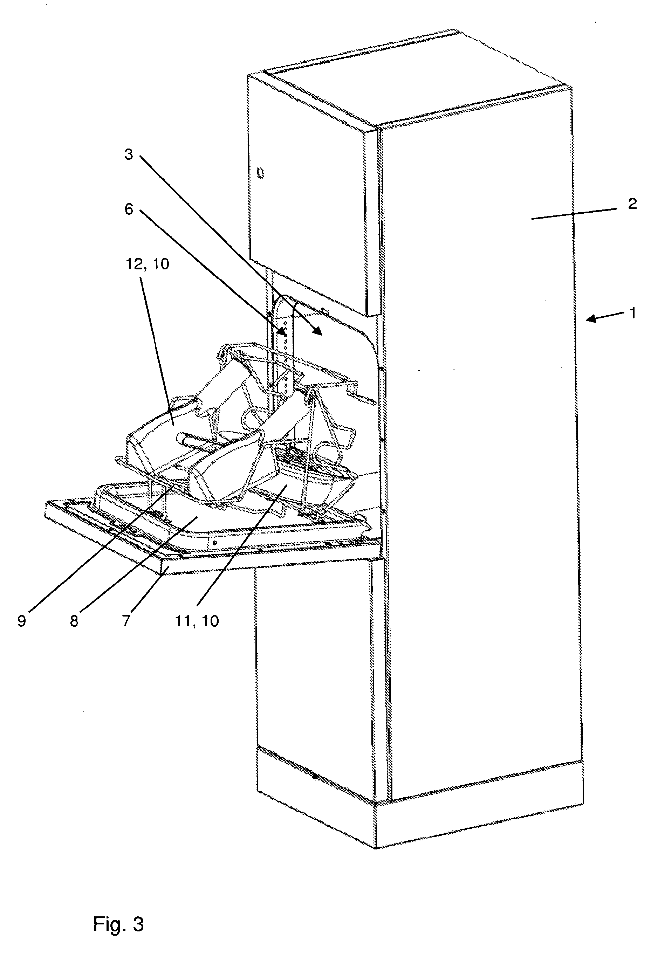

- Fig.3: eine Spüleinrichtung nach Fig. 1 mit geöffneter Tür und
- Fig. 4: in schematischer Darstellung die erfindungsgemäße Verfahrensdurchführung

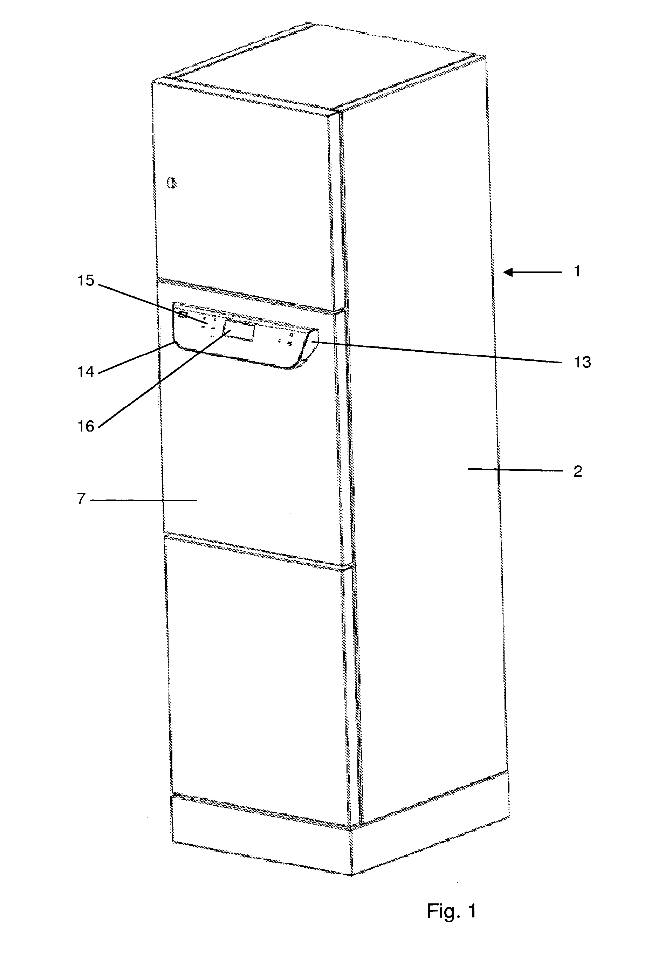

Fig. 1 zeigt eine Spüleinrichtung 1, diese dient der Reinigung von Spülgütern 10, insbesondere von Steckbecken 11, Urinflaschen 12 und/oder dergleichen. Die Spüleinrichtung 1 kann deshalb auch als Steckbeckenreinigungsautomat bezeichnet werden.

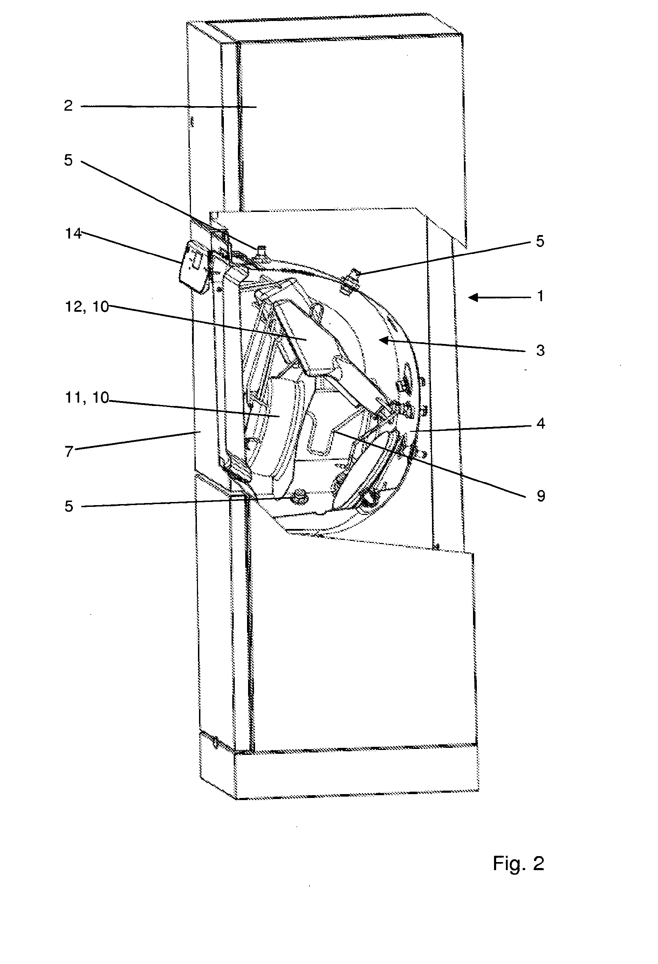

Die Spüleinrichtung 1 verfügt über ein Gehäuse 2. Dieses stellt einen Einsatz 4 bereit, wie insbesondere die Darstellung nach Fig. 2 erkennen lässt. Der Einsatz 4 definiert den eigentlichen Reinigungsraum 3.

Der Reinigungsraum 3 ist über eine Beschickungsöffnung 6 zugänglich. Diese ist mittels einer verschwenkbar ausgebildeten Tür 7 fluiddicht verschließbar.

Die Tür 7 ist türaußenseitig mit einer Bedieneinrichtung 14 ausgestattet. Diese stellt einen Griff 13 bereit, der verwenderseitig zur Betätigung der Tür 7 ergriffen werden kann. Die Bedieneinrichtung 14 stellt des Weiteren Bedienelemente 15 sowie ein Display 16 bereit. Die Bedienelemente 15 dienen insbesondere dazu, ein mit der Spüleinrichtung 1 durchzuführendes Spül- beziehungsweise Reinigungsprogramm auszuwählen und/oder vorzugeben. Über das Display 16 kann das gewählte Reinigungsprogramm angezeigt werden. Auch können mittels des Displays 16 Statusanzeigen zur Anzeige gebracht werden.

Auf der Türinnenseite 8 der Tür 7 ist eine Spülgutaufnahme in Form eines Gestells 9 angeordnet. Bei dem Gestell 9 kann es sich beispielsweise um einen Drahtkorb handeln.

Nach einer ordnungsgemäßen Bestückung des Gestells 9 mit beispielsweise Steckbecken 11 1 und/oder Urinflaschen 12 ist die Tür 7 in die Verschlussstellung nach Fig. 2 bedienerseitig zu verschwenken. Infolge dieser Verschwenkbewegung schwenkt das türinnenseitig vom Gestell 9 getragene Spülgut 10 in den vom Einsatz 4 definierten Reinigungsraum 3 ein, wie dies die teilgeschnittene Seitenansicht nach Fig. 2 erkennen lässt.

Zum Zwecke der Beaufschlagung des Spülguts 10 mit Wasser und/oder Reinigungsmitteln verfügt der Einsatz 4 über eine Mehrzahl von Düsen 5.

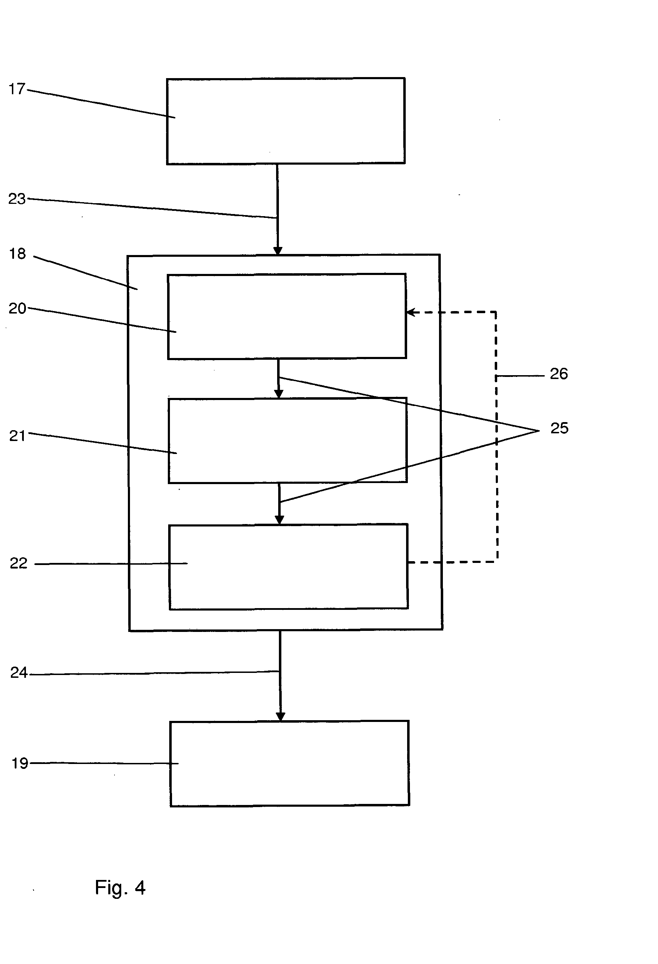

Die erfindungsgemäße Verfahrensdurchführung ergibt sich aus der schematischen Blockdarstellung nach Fig. 4.

In einem ersten Verfahrensschritt erfolgt eine mechanische Reinigung 17. Diese erfolgt beispielsweise mittels Wasser, das als sogenanntes Schwallwasser über die Düsen 5 in den Reinigungsraum 3 eingebracht wird. Je nach Verschmutzungsgrad der zu reinigenden Spülgüter 10 kann dieser Wassereintrag unter Druck erfolgen.

Die Beaufschlagung des Spülgutes 10 mit Wasser führt zu einer ersten Reinigung des Spülguts 10. Das in den Reinigungsraum 3 eingebrachte Wasser wird mitsamt der abgelösten Verschmutzungen über einen nicht näher dargestellten Siphon einer ebenfalls nicht näher dargestellten Abwasserleitung zugeführt. Eine Umwälzung des Spülwassers innerhalb des Reinigungsraums 3 findet nicht statt.

Wie dies der Pfeil 23 nach Fig. 4 erkennen lässt, folgt dem Verfahrensschritt der mechanischen Reinigung 17 der Verfahrensschritt der chemischen Reinigung 18. Die chemische Reinigung erfolgt unter Einsatz von additiv verwendeten chemischen Reinigungsmitteln. Zu diesem Zweck kommt ein Gemisch aus Wasser einerseits und chemischen Reinigungsmitteln andererseits zum Einsatz, das gleichfalls über die Düsen 5 auf das zu reinigende Spülgut 10 aufgebracht wird.

Im Anschluss an den Verfahrensschritt der chemischen Reinigung 18 folgt der Verfahrensschritt der thermischen Desinfektion 19, wie dies durch den Pfeil 24 symbolisiert ist.

Der Verfahrensschritt der thermischen Desinfektion 19 erfolgt bevorzugterweise mittels entspanntem Wasserdampf. Zu diesem Zweck verfügt die Spüleinrichtung 1 über einen in den Fign. nicht näher dargestellten Dampferzeuger. Der vom Dampferzeuger erzeugte Dampf wird in den Reinigungsraum 3 eingeleitet, wo es dann zur thermischen Desinfektion des Spülgutes 10 kommt. Nach einer Abkühlung des durch die thermische Desinfektion aufgewärmten Spülgutes 10 kann dieses vom Verwender dem Reinigungsraum 3 entnommen werden.

Der Verfahrensschritt der chemischen Reinigung 18 ist erfindungsgemäß dreistufig ausgebildet.

Gemäß einer ersten Stufe 20 wird das Spülgut 10 mit einem Gemisch aus Wasser und einem chemischen Reinigungsmittel beaufschlagt. Alsdann folgt in einer zweiten Stufe 21 ein Einwirken des auf dem Spülgut befindlichen Gemisches aus Wasser und Reinigungsmittel für eine vorgebbare Zeitspanne. Infolge dieser Einwirkung kommt es zur Auf- und/oder Ablösung der am Spülgut 10 anhaftenden Verschmutzungen. In einer dritten und letzten Stufe 22 wird ein Abspülen des Spülgutes mittels Frischwasser durchgeführt.

Die einzelnen Stufen 20, 21, und 22 des Verfahrensschrittes der chemischen Reinigung 18 werden aufeinander nachfolgend durchgeführt, was die Pfeile 25 nach Fig. 4 symbolisieren.

Je nach Verschmutzungsgrad der zu reinigenden Spülgüter 10 kann vorgesehen sein, die Verfahrensstufen 20, 21 und 22 mehrfach durchzuführen. Diese Verfahrensalternative ist durch den in Fig. 4 durch Strichlinie eingezeichneten Pfeil 26 wiedergegeben.

## Patentansprüche

1. Verfahren zur Reinigung von Steckbecken (11), Urinflaschen (12) als Spülgüter (10), bei dem eine mechanische Reinigung (17), anschließend eine chemische Reinigung (18) und abschließend eine thermische Desinfektion (19) durchgeführt wird, wobei das gesamte Verfahren nach dem Durchflussprinzip arbeitet,
**dadurch gekennzeichnet,**
**dass** der Verfahrensschritt der chemischen Reinigung dreistufig in der Art durchgeführt wird, dass das Spülgut (10) in einer ersten Stufe (20) mit einem Gemisch aus Wasser und einem chemischen Reinigungsmittel beaufschlagt wird, in einer zweiten Stufe (21) ein Einwirken des auf dem Spülgut (10) befindlichen Gemisches aus Wasser und Reinigungsmittel für eine vorgebbare Zeitspanne erfolgt und in einer dritten Stufe (22) ein Abspülen des Spülguts (10) mit Frischwasser durchgeführt wird.

2. Verfahren nach Anspruch 1,
**dadurch gekennzeichnet,**
**dass** die Behandlungsstufen (20, 21, 22) des Verfahrensschritts der chemischen Reinigung (18) mehrfach aufeinander nachfolgend durchgeführt werden.

3. Verfahren nach Anspruch 1 oder 2,
**dadurch gekennzeichnet,**
**dass** zur Herstellung des Gemisches aus Wasser und einem Reinigungsmittel ein Wassertank dient, in den eine vorgebbare Menge eines Reinigungsmittels und eine vorgebbare Menge an Wasser eingefüllt wird.

4. Verfahren nach einem der vorhergehenden Ansprüche,
**dadurch gekennzeichnet,**
**dass** der Wassertank während der Verfahrensstufe (21) des Einwirkens mit Frischwasser befüllt wird.

5. Verfahren nach einem der vorhergehenden Ansprüche;
**dadurch gekennzeichnet,**
**dass** das Frischwasser erwärmt wird.

## Claims

1. Method for cleaning bedpans (11) and urine bottles (12) as washware (10), in which mechanical cleaning (17), then chemical cleaning (18) and finally thermal disinfection (19) are carried out, the entire method operating according to the continuous flow principle,
**characterised in that**
the method step of chemical cleaning is carried out in three stages such that, in a first stage (20), a mixture of water and a chemical cleaning agent is applied to the washware (10), in a second stage (21), the water and cleaning agent mixture on the washware (10) act for a predetermined period of time, and in a third stage (22), the washware (10) is rinsed with fresh water.

2. Method according to claim 1,
**characterised in that**
the treatment stages (20, 21, 22) of the method step of chemical cleaning (18) are carried out multiple times in succession.

3. Method according to either claim 1 or claim 2,
**characterised in that**
a water tank is used to produce the water and cleaning agent mixture, into which tank a predeterminable amount of cleaning agent and a predeterminable amount of water are filled.

4. Method according to any of the preceding claims
**characterised in that**
the water tank is filled with fresh water during the action method step (21).

5. Method according to any of the preceding claims;
**characterised in that**
the fresh water is heated.

## Revendications

1. Procédé de nettoyage de bassins (11) et d'urinaux (12) en tant que produits à rincer (10), dans lequel sont effectués un nettoyage mécanique (17), puis un nettoyage chimique (18) et enfin une désinfection thermique (19), la totalité du procédé fonctionnant selon le principe d'écoulement,
**caractérisé en ce que**
l'étape de procédé du nettoyage chimique est réalisée en trois stades de telle sorte que le produit à rincer (10) est, dans un premier stade (20), alimenté avec un mélange d'eau et d'un détergent chimique, dans un deuxième stade (21) un action du mélange d'eau et de détergent présent sur le produit à rincer (10) s'exerce pendant une durée pouvant être prédéfinie, et dans un troisième stade un rinçage du produit à rincer (10) est effectué avec de l'eau fraîche.

2. Procédé selon la revendication 1,
**caractérisé en ce que**
les stades de traitement (20, 21, 22) de l'étape de procédé du nettoyage chimique (18) sont effectués plusieurs fois les uns à la suite des autres.

3. Procédé selon la revendication 1 ou 2,
**caractérisé en ce que**,
pour la réalisation du mélange d'eau et d'un détergent, on utilise un réservoir d'eau dans lequel on verse une quantité de détergent pouvant être prédéfinie et une quantité d'eau pouvant être prédéfinie.

4. Procédé selon l'une quelconque des revendications précédentes,
**caractérisé en ce que**,
pendant le stade de procédé (21) d'action, le réservoir d'eau est rempli avec de l'eau fraîche.

5. Procédé selon l'une quelconque des revendications précédentes,
**caractérisé en ce que**
l'eau fraîche est réchauffée.
